# FASCICULE DE BREVET EUROPEEN

(11) **EP 1 556 496 B1**
(45) Date de publication et mention de la délivrance du brevet: **25.07.2007**
(21) Numéro de dépôt: 03778470.9
(22) Date de dépôt: 27.10.2003
(51) Int. Cl.: C12N 15/90

(54) **PROCEDE POUR INSERER UN ACIDE NUCLEIQUE D INTERET DANS UNE CELLULE PROCARYOTE OU EUCARYOTE PAR RECOMBINAISON HOMOLOGUE**
VERFAHREN ZUR EINFÜHRUNG EINER NUCLEINSÄURE IN EINE PROKARYONTISCHE ODER EUKARYOTISCHE ZELLE DURCH HOMOLOGE REKOMBINATION
METHOD FOR INSERTING A NUCLEIC ACID OF INTEREST INTO A PROKARYOTIC OR EUKARYOTIC CELL BY HOMOLOGOUS RECOMBINATION

(30) Priorité: 28.10.2002 FR 0213474
(43) Date de publication de la demande: 27.07.2005
(73) Titulaire: CENTRE NATIONAL DE LA RECHERCHE SCIENTIFIQUE (CNRS), 75794 Paris Cedex 16 (FR)
(72) Inventeur: FUCHS, Robert, P., P., F-13009 Marseille (FR); BICHARA, Marc, Bernard, F-67550 Vendenheim (FR)
(74) Mandataire: Goulard, Sophie
(86) Numéro de dépôt international: PCT/FR2003/003188
(87) Numéro de publication internationale: WO 2004/039983

(56) Documents cités:
- WO-A-01/68882
- HINDS J ET AL: "Enhanced gene replacement in mycobacteria." MICROBIOLOGY (READING, ENGLAND) ENGLAND MAR 1999, vol. 145 ( Pt 3), mars 1999 (1999-03), pages 519-527, XP002246130 ISSN: 1350-0872
- MAHER V M ET AL: "MUTATIONS AND HOMOLOGOUS RECOMBINATION INDUCED BY N-SUBSTITUTED ARYL COMPOUNDS IN MAMMALIAN CELLS" ENVIRONMENTAL SCIENCE RESEARCH, 1990, pages 149-156, XP008018516 ISSN: 0090-0427
- POSFAI G ET AL: "Markerless gene replacement in escherichia coli stimulated by a double-strand break in the chromosome" NUCLEIC ACIDS RESEARCH, OXFORD UNIVERSITY PRESS, SURREY, GB, vol. 27, no. 22, 15 novembre 1999 (1999-11-15), pages 4409-4415, XP002963851 ISSN: 0305-1048
- NAIRN R S ET AL: "Transformation depending on intermolecular homologous recombination is stimulated by UV damage in transfected DNA" MUTATION RESEARCH 1988 NETHERLANDS, vol. 208, no. 3-4, 1988, pages 137-141, XP002281444 ISSN: 0165-7992
- DIANOV G L ET AL: "THE CHEMICAL MUTAGEN DIMETHYL SULFATE INDUCED HOMOLOGOUS RECOMBINATION OF PLASMID DNA BY INCREASING THE BINDING OF RECA PROTEIN TO DUPLEX DNA" MUTATION RESEARCH, vol. 249, no. 1, 1991, pages 189-193, XP002281445 ISSN: 0027-5107

## Description

### DOMAINE DE L'INVENTION

La présente invention se rapporte au domaine de l'insertion ciblée d'un acide nucléique d'intérêt dans une localisation choisie d'un acide nucléique génomique présent dans un chromosome contenu dans une cellule procaryote ou eucaryote.

### ETAT DE LA TECHNIQUE

La mise au point de techniques efficaces et reproductibles en vue de l'insertion ciblée d'un acide nucléique d'intérêt à un endroit choisi de l'ADN d'un chromosome fait actuellement l'objet de nombreux travaux, en particulier de travaux relatifs à la mise au point de techniques de thérapies géniques cellulaires somatiques, qui visent à prévenir ou traiter des pathologies humaines ou animales associées à un déficit d'origine génétique.

Ces techniques sont utiles pour traiter des déficits génétiques provoqués par la mutation du gène sauvage initial. On peut citer par exemple le-gène *cftr* dont certaines mutations provoquent la maladie de la mucoviscidose, aussi désignée fibrose kystique.

L'insertion ciblée d'un acide nucléique au sein d'un ADN chromosomique est également utile dans le cadre de procédés de production d'animaux transgéniques modèles, notamment afin d'étudier les effets physiologiques de la surexpression (animaux « knock-in ») ou au contraire du blocage de l'expression (animaux « knock-out ») d'un gène d'intérêt, notamment en vue de mettre au point de nouveaux médicaments.

Différentes techniques d'insertion ciblée d'un acide nucléique d'intérêt à un endroit précis et prédéterminé de l'ADN d'un chromosome sont d'ores et déjà disponibles. De nombreuses techniques ont été détaillées notamment dans un article de revue de CAPECCHI (1989). Par exemple, on a décrit des techniques d'insertion ciblée d'ADN par recombinaison homologue par co-transformation de cellules avec deux vecteurs distincts, respectivement un vecteur contenant un gène de sélection et un vecteur utilisé pour la recombinaison homologue, ces techniques étant utilisées en particulier pour l'interruption d'un gène cible (Reid et al, 1991).

On a aussi décrit des systèmes de recombinaison homologue à deux vecteurs, respectivement (i) un premier vecteur destiné à être intégré dans le génome cible et fournissant un site unique de recombinaison homologue et (ii) un second vecteur comprenant la séquence d'intérêt à insérer au niveau du site unique de recombinaison préalablement intégré (brevet US N° 5,998, 144).

D'autres travaux concernent des systèmes de recombinaison homologue dans lesquels trois partenaires moléculaires, respectivement (i) un fragment d'ADN donneur double brin, (ii) un premier ADN de liaison double-brin et (iii) un second ADN de liaison double brin (brevet américain N° 6,207,442).

L'utilisation de vecteurs rétro-viraux pour réaliser l'insertion ciblée d'ADN par recombinaison homologue a également été décrite (brevet américain N° 6,281,000), ainsi que l'utilisation de vecteurs comprenant deux gènes marqueurs de sélection, respectivement un marqueur de sélection négative et un marqueur de sélection positive (brevet américain N° 6,284,541).

On connaît aussi des techniques de recombinaison homologue qui tirent parti de la formation d'une structure du type triple hélice d'ADN à l'endroit du chromosome préalablement choisi pour l'insertion de l'ADN d'intérêt, comme cela est décrit notamment dans les brevets américains US N° 5,962,426 et US N° 6,303,376.

On a aussi décrit des techniques d'insertion de gènes comportant une étape de transfection des cellules cibles avec un vecteur qui ne se réplique pas dans ces cellules, aussi appelé « vecteur suicide », le vecteur suicide étant au préalable soumis à une exposition au rayonnement ultraviolet (Hinds et al., 1999).

Toutefois, la pratique des techniques de recombinaison homologue ci-dessus, en vue d'insérer de manière ciblée un acide nucléique dans un chromosome, illustre le fait que les séquences d'ADN d'intérêt exogènes transférées dans des cellules, en particulier des cellules eucaryotes, ne subissent des évènements de recombinaison homologue, avec les séquences endogènes homologues de l'hôte cellulaire, qu'à des fréquences très basses de recombinaison, ce qui nécessite le recours à la transfection, puis la sélection, d'un très grand nombre de cellules afin de produire au moins un clone de cellules recombinantes pour lequel la séquence d'ADN d'intérêt a effectivement été insérée à l'endroit choisi du génome.

De plus, pour certaines des techniques ci-dessus, le ou les vecteurs d'ADN utilisés pour réaliser la recombinaison homologue n'est (ne sont) pas éliminé (s) de l'hôte cellulaire, ce qui entraîne de nombreux inconvénients, notamment du fait que ces vecteurs d'ADN comprennent en général des gènes de sélection consistant en des gènes de résistance à divers antibiotiques, susceptibles de diffuser subséquemment dans l'hôte procaryote ou eucaryote recombiné.

De façon générale, les techniques de ciblage de gènes dans les organismes eucaryotes supérieurs se- heurtent au fait que les évènements de recombinaison non homologue sont de 1000 à 100 000 fois plus fréquents que les évènements de recombinaison homologue. L'absence de technique permettant d'augmenter efficacement la fréquence de recombinaison homologue a orienté les recherches vers le développement de systèmes d'enrichissement en clones recombinants homologues basés sur des sélections génétiques dont l'objet est d'éliminer les clones recombinants chez lesquels se sont réalisés des évènements de recombinaison non homologue. Cependant, du fait de la fréquence très faible des évènements de recombinaison homologue, et malgré la forte pression de sélection exercée sur les clones cellulaires recombinants, il est souvent très difficile d'obtenir les clones recombinants recherchés, a fortiori de manière reproductible.

### DESCRIPTION DE L'INVENTION

Le demandeur s'est attaché à mettre au point un procédé permettant l'insertion ciblée d'un ADN d'intérêt dans le chromosome d'une cellule, par recombinaison homologue, avec une haute fréquence des évènements de recombinaison homologue, et à l'issue duquel le vecteur porteur de la séquence d'intérêt à insérer de manière ciblée, qui peut comprendre des séquences indésirables tels que des gènes de résistance aux antibiotiques, est éliminé.

Il est montré selon l'invention que la mise en contact d'un vecteur d'ADN comprenant un acide nucléique d'intérêt avec un agent mutagène qui crée, dans ledit vecteur d'ADN, des lésions susceptibles d'interférer avec sa réplication dans la cellule, permet l'insertion ciblée, par un événement de recombinaison homologue, de cet acide nucléique d'intérêt dans une localisation choisie dans le génome de ladite cellule, avec une haute fréquence de l'événement de recombinaison homologue.

Simultanément, les parties du vecteur d'ADN, autres que l'acide nucléique d'intérêt qui est initialement inclus dans ce dernier, sont éliminées de la cellule hôte procaryote ou eucaryote recombinée.

Ainsi, selon l'invention, on a montré que l'utilisation d'un vecteur d'ADN comprenant un acide nucléique d'intérêt, ledit vecteur se répliquant dans les cellules procaryotes ou eucaryotes cibles, permet, lorsque ce vecteur d'ADN est traité avec un agent mutagène préalablement à l'étape de transfection dans les cellules, l'obtention d'une haute fréquence d'évènements de recombinaison homologues aboutissant à l'insertion ciblée dudit acide nucléique d'intérêt à un endroit choisi du génome cellulaire.

Sans vouloir être lié par une quelconque théorie, le demandeur pense que la haute fréquence des évènements de recombinaison homologue, et donc la haute fréquence d'insertion de l'acide nucléique d'intérêt dans le génome des cellules cibles, est due à la création, au moment de la réplication du vecteur d'ADN dans les cellules transfectées, d'extrémités d'ADN néosynthétisé hautement recombinogènes. Ainsi, grâce au traitement du vecteur d'ADN par l'agent mutagène, la réplication du vecteur dans la cellule est bloquée au niveau des lésions provoquées par cet agent mutagène sur l'ADN du vecteur, ce qui aboutit à la production de fragments d'ADN nouvellement synthétisé dont les extrémités sont capables de se recombiner avec l'ADN cible chromosomique, par des évènements de recombinaison homologue non réciproque du vecteur vers le chromosome cible dans la cellule.

C'est la raison pour laquelle les agents mutagènes selon l'invention sont choisis parmi les agents qui bloquent la réplication de l'ADN dans la cellule procaryote ou eucaryote et qui créent ainsi des structures recombinogènes au sein de l'ADN traité par lesdits agents mutagènes.

Par structure recombinogène, on entend une ou plusieurs régions de l'ADN traité dans lesquelles la structure double brin de l'ADN est affectée, par exemple par création d'un mésappariement des bases, ce qui inclut une éventuelle coupure de l'un des brins d'ADN. Ces structures recombinogènes d'ADN sont créées lors de l'interruption de la synthèse d'ADN du fait du traitement initial avec l'agent mutagène choisi et incluent de façon non exhaustive des cassures simple brin, des brèches simple brin ainsi que des cassures double brin.

En particulier, il est montré selon l'invention que la misé en contact du vecteur d'ADN comprenant un acide nucléique d'intérêt avec l'agent mutagène N-acétoxy-2-acétylaminofluorène (N-AcO-AAF) permettait l'insertion ciblée de cet ADN d'intérêt dans une localisation choisie de l'ADN d'un chromosome contenu dans une cellule, par recombinaison homologue non réciproque du vecteur vers le chromosome, avec une haute fréquence d'occurrence de l'événement de recombinaison homologue.

La fixation des molécules de N-AcO-AAF sur le polynucléotide comprenant l'ADN d'intérêt permet l'obtention d'évènements de recombinaison homologue non réciproque dans le sens du- vecteur d'ADN vers le chromosome, avec une fréquence qui peut être supérieure à 0,05 événements de recombinaison homologue par cellule transfectée.

L'invention a donc pour objet l'utilisation d'un agent mutagène bloquant la réplication de l'ADN dans la cellule pour insérer *in vitro* un acide nucléique d'intérêt au sein d'une séquence nucléotidique prédéterminée présente dans un chromosome contenu dans une cellule procaryote ou eucaryote, ledit acide nucléique d'intérêt étant, préalablement à son insertion, inclus dans un vecteur d'ADN qui se réplique dans ladite cellule hôte procaryote ou eucaryote.

Un agent mutagène bloquant la réplication de l'ADN dans la cellule englobe, selon l'invention, tout composé chimique naturel ou de synthèse, ou encore un rayonnement ultraviolet (UV), dont l'activité de blocage de la réplication de l'ADN peut être déterminée par toute technique connue de l'homme du métier, par exemple la technique décrite par FUCHS (1984), qui utilise l'activité exonucléase 5'→ 3' de l'ADN polymérase du phage T4, laquelle est bloquée au voisinage des bases chimiquement modifiées.

Avantageusement, l'agent mutagène est choisi parmi le N-AcO-AAF, les agents alkylants, le benzo(a)pyrene-diol-époxyde (BPDE), ou encore un rayonnement U.V.

Lorsque l'agent mutagène consiste en un rayonnement UV, l'ADN à traiter est avantageusement irradié par une source de rayons UV immédiatement avant l'utilisation de cet ADN pour transfecter les cellules. Par exemple, l'ADN peut être ajusté à la concentration de 100µg/ml dans un tampon TE (pH 8,0) puis irradié à la température de 20°C-25°C à la puissance de 1,8 J.m⁻².s⁻¹ à l'aide d'une lampe germicide, par exemple la lampe référencée G8T5 (General Electric).

Préférentiellement, l'agent mutagène est le N-acétoxy-2-acétylaminofluorène (N-AcO-AAF).

Le composé N-AcO-AAF est connu dans l'état de la technique en tant qu'agent mutagène. Le composé N-AcO-AAF a notamment été utilisé dans le cadre de travaux académiques relatifs à l'étude du mécanisme de réparation de l'ADN des cellules bactériennes. Lorsque le composé N-AcO-AAF est mis en contact avec un plasmide bactérien qui est subséquemment transfecté dans des cellules d'*Escherichia coli,* on observe une induction des mécanismes intracellulaires de réparation de l'ADN, lesquels par des étapes d'excision puis d'élongation de l'ADN clivé, permettent la survie du plasmide dans les cellules de *E.coli* (Schmid et al., 1982).

Il a aussi été montré que la fréquence de certains évènements de recombinaison homologue, du chromosome vers le plasmide, peut être augmentée lorsque le plasmide est préalablement traité avec le N-AcO-AAF, dans un système cellulaire *Escherichia coli.*

Ainsi, Luisi-DeLuca et al. (1984) utilisent un plasmide portant le gène lacY⁺ dans un hôte bactérien de phénotype LacY⁻. Après transformation de la cellule bactérienne par le plasmide, la majorité des bactéries transformées sont de phénotypes Lac⁻. Les transformants Lac⁻ obtenus proviennent d'un transfert de l'allèle lacY⁻ du chromosome vers le plasmide.

Maher et al. (1990) avaient aussi montré que le N-AcO-AAF induit des évènements de recombinaison homologue intrachromosomique entre des gènes présents dans des cellules de souris, dans le cadre d'une étude académique sur les mécanismes d'induction de cancers par des agents mutagènes.

Il a été montré pour la première fois selon l'invention qu'un agent mutagène tel que défini ci-dessus, en particulier le composé N-AcO-AAF, lorsqu'il est mis en contact avec un vecteur d'ADN réplicatif comprenant un ADN d'intérêt à insérer de manière ciblée dans le chromosome d'une cellule procaryote ou eucaryote, est capable d'induire un transfert de l'ADN d'intérêt dudit vecteur, qui peut être par exemple un plasmide, vers le chromosome, et ceci avec une fréquence très élevée des évènements de recombinaison homologue non réciproques du polynucléotide vers le chromosome.

L'invention a donc également pour objet un procédé pour insérer *in vitro* un acide nucléique d'intérêt inclus initialement dans un vecteur d'ADN, au sein d'une séquence nucléotidique prédéterminée présente dans un chromosome contenu dans une cellule procaryote ou eucaryote, caractérisé en ce qu'il comprend les étapes de :
a) mettre en contact le vecteur d'ADN comprenant l'acide nucléique d'intérêt, et qui se réplique dans ladite cellule procaryote ou eucaryote, avec un agent mutagène bloquant la réplication de l'ADN dans la cellule ;
b) transfecter des cellules procaryotes ou eucaryotes avec le vecteur d'ADN tel qu'obtenu à la fin de l'étape a) ;
c) sélectionner les cellules procaryotes ou eucaryotes pour lesquelles l'acide nucléique d'intérêt a été intégré dans la séquence nucléotidique prédéterminée.
   De préférence, le procédé ci-dessus comprend en outre l'étape suivante :
d) sélectionner, parmi les cellules procaryotes ou eucaryotes sélectionnées à l'étape c), les cellules dans lesquelles les séquences du vecteur d'ADN, autres que celles de l'acide nucléique d'intérêt, ont été éliminées.

Avantageusement, le composé N-AcO-AAF est préparé par synthèse chimique à partir du Nitrofluorène, selon des techniques connues de l'homme du métier, comme par exemple la technique décrite par LEFEVRE et al. (1978).

Le Nitrofluorène de départ, ainsi que le composé N-AcO-AAF final, peuvent être obtenus notamment auprès de la Société AMERSHAM.

On a montré selon l'invention, que la fréquence des évènements de recombinaison homologue non réciproque du vecteur d'ADN, par exemple un plasmide, vers le chromosome, augmente avec un nombre croissant de lésions ou de bases chimiquement modifiées provoquées par l'agent mutagène dans ledit vecteur d'ADN.

Par exemple, on a montré selon l'invention que la fréquence des évènements de recombinaison homologue non réciproque du vecteur d'ADN, par exemple un plasmide, vers le chromosome, augmente avec un nombre croissant de molécules d'agent mutagène, comme le N-AcO-AAF, fixés sur ledit vecteur d'ADN.

Ainsi, la fréquence d'événements de recombinaison homologue passe de 5,61 x 10⁻⁴ pour dix molécules de N-AcO-AAF par molécule du vecteur d'ADN comprenant l'acide nucléique d'intérêt, à plus de 600 x 10⁻⁴ pour 67 molécules de N-AcO-AAF par molécule du vecteur d'ADN comprenant l'acide nucléique d'intérêt.

Des fréquences accrues de recombinaison homologue non réciproque du vecteur d'ADN vers le chromosome peuvent être obtenues avec des rapports encore plus élevés de lésions de l'ADN ou de nombre de molécules d'agent mutagène, comme le N-AcO-AAF, par molécule dudit vecteur.

Ainsi, le nombre de lésions de l'ADN ou de molécules d'agent mutagène par molécule du vecteur d'ADN comprenant l'acide nucléique d'intérêt peut dépasser 100/1.

Avantageusement à l'étape a) du procédé, la concentration finale de l'agent mutagène utilisé est adaptée à la fixation d'au moins 10 molécules d'agent mutagène par molécule du vecteur d'ADN. Préférentiellement, la concentration finale de l'agent mutagène utilisé est adaptée à la fixation d'au moins 50 molécules de cet agent mutagène par molécule vecteur d'ADN.

Selon l'invention, le nombre de molécules de l'agent mutagène, en particulier de N-Aco-AAF, par molécule du vecteur d'ADN comprenant l'acide nucléique d'intérêt est au minimum de 10/1 et peut aller au-delà de 100/1, par exemple jusqu'à 200/1.

Avantageusement, le nombre de molécules de l'agent mutagène par molécule du vecteur d'ADN comprenant l'acide nucléique d'intérêt est compris entre 10/1 et 100/1, le rapport optimal nombre de molécules de l'agent mutagène/molécule dudit vecteur d'ADN étant choisi en fonction de la fréquence de recombinaison homologue non réciproque du vecteur vers le chromosome désirée.

Afin d'obtenir le rapport molaire désiré agent mutagène/vecteur d'ADN, l'homme du métier peut faire varier (i) les concentrations relatives dudit agent mutagène et dudit vecteur qui sont mis en contact à l'étape a) du procédé et/ou (ii) la durée de l'étape a) de mise en contact dudit agent mutagène avec ledit vecteur.

De préférence, pour un vecteur d'ADN donné, d'une taille donnée, on modifie le rapport molaire agent mutagène/vecteur d'ADN en faisant varier la durée de l'étape a) de mise en contact entre ledit agent mutagène et ledit vecteur.

De même, pour l'obtention d'un rapport molaire agent mutagène/vecteur donné, on fait varier la durée de l'étape a) en fonction de la taille du vecteur d'ADN considéré, la durée de l'étape a) étant d'autant plus longue que la taille du vecteur d'ADN est grande.

L'homme du métier peut aisément déterminer si, à la fin de l'étape a) du procédé, le rapport molaire agent mutagène/vecteur d'ADN désiré a été atteint, selon des techniques conventionnelles.

Par exemple, à la fin de l'étape a), une fraction aliquote du mélange agent mutagène/vecteur est prélevée et les molécules libres d'agent mutagène sont éliminées, par exemple par précipitation de l'ADN puis filtration sur filtre de nitrocellulose.

Puis, on détermine respectivement le nombre de moles du vecteur et le nombre de moles d'agent mutagène fixées sur ledit vecteur afin d'établir le rapport agent mutagène/vecteur qui a été atteint.

Le nombre de moles du vecteur d'ADN contenues dans la fraction aliquote peut être classiquement déterminé par spectrophotométrie UV à la longueur d'onde de 260 nanomètres.

Le nombre de moles de l'agent mutagène fixées sur ce vecteur d'ADN peut être déterminé par mesure de radioactivité, par exemple lorsque, pour les essais, on a utilisé un agent mutagène marqué avec un isotope radioactif, tel que ³[H].

Une molécule d'agent mutagène se fixe sur une base du veteur. Le résultat peut donc être exprimé en pourcentage de bases du vecteur d'ADN qui ont été modifiées par l'agent mutagène.

Par exemple, on a déterminé selon l'invention, lorsqu'on a traité un vecteur, par exemple un plasmide, d'une longueur de 5000 paires de bases, que les rapports molaires agent mutagène/vecteur étaient respectivement de 13 (0,26% de bases modifiées) pour une durée de l'étape a) de 4 minutes, 28 (0,36% de bases modifiées) pour une durée de 8 minutes, et 56 (1,12% de bases modifiées) pour une durée de 20 minutes, après mise en contact de 60 µg du vecteur d'ADN en solution avec 1,2 µg de N-AcO-AAF.

Pour la mise en oeuvre de l'étape a) du procédé, le vecteur comprenant l'acide nucléique d'intérêt est avantageusement en suspension dans un tampon salin, de préférence un tampon citrate, éventuellement additionné d'éthanol.

L'agent mutagène est en solution dans un solvant approprié. Dans le cas du N-AcO-AAF, on utilise de préférence l'éthanol.

Pour un essai donné, l'homme du métier adaptera le rapport molaire agent mutagène/vecteur d'ADN, par de simples essais de routine, jusqu'à l'obtention de la fréquence optimale d'évènements de recombinaison homologue recherchée.

Par « vecteur d'ADN » au sens de la présente invention, on entend une molécule d'ADN circulaire ou linéaire qui est indifféremment sous la forme simple-brin ou double-brin, et qui se réplique dans la cellule hôte procaryote ou eucaryote dans laquelle ce vecteur d'ADN doit être transfecté.

De préférence, le vecteur d'ADN selon l'invention est un vecteur susceptible d'être répliqué dans un hôte cellulaire choisi, par exemple une cellule bactérienne et encore plus spécifiquement une cellule d'*Escherichia coli,* afin d'en produire de grandes quantités dans les cellules hôtes. Une fois que le vecteur d'ADN est obtenu en une quantité suffisante pour la réalisation de l'étape de transfection dans la cellule hôte choisie, ce vecteur est utilisé comme matériel de départ pour l'insertion ciblée, dans le génome cellulaire, de l'acide nucléique d'intérêt qui est inséré dans celui-ci.

Un vecteur d'ADN pour la mise en oeuvre du procédé selon l'invention comprend, outre l'acide nucléique d'intérêt, également au moins une origine de réplication fonctionnelle dans la cellule hôte dans laquelle il doit être transfecté en vue de l'insertion ciblée, par recombinaison homologue, de l'acide nucléique d'intérêt inséré dans ce vecteur. Un vecteur d'ADN selon l'invention comprend 1, 2, 3, 4 ou 5 origines de réplications fonctionnelles dans la cellule hôte dans laquelle il doit être transfecté en vue de l'insertion ciblée de l'acide nucléique d'intérêt dans la cellule hôte procaryote ou eucaryote choisie.

Lorsque la cellule hôte à transfecter est une cellule procaryote, par exemple une cellule hôte bactérienne, et encore plus spécifiquement une cellule de *Escherichia coli,* le vecteur d'ADN comprend au moins une origine de réplication fonctionnelle dans cette cellule procaryote, par exemple une origine de réplication fonctionnelle chez *Escherichia coli.*

Lorsque la cellule hôte à transfecter est une cellule eucaryote, par exemple une cellule hôte de mammifère, et encore plus spécifiquement une cellule humaine, le vecteur d'ADN comprend au moins une origine de réplication fonctionnelle dans cette cellule eucaryote, par exemple une origine de réplication fonctionnelle dans des cellules humaines.

Avantageusement, un tel vecteur comprend aussi la séquence d'un gène marqueur, par exemple un gène de résistance aux antibiotiques, permettant la sélection des cellules hôtes, notamment des cellules hôtes de *E.coli,* qui ont été transfectées avec ledit vecteur, ces cellules transfectées permettent, par leur culture dans un milieu de culture approprié, l'obtention de grandes quantités du vecteur.

Avantageusement, un tel vecteur comprend aussi la séquence d'un gène marqueur fonctionnel dans la cellule hôte dans laquelle on recherche l'insertion de l'acide nucléique d'intérêt par recombinaison homologue, la détection de l'expression dudit gène marqueur permettant la sélection positive ou négative des cellules hôtes ayant été effectivement transfectées avec ce vecteur.

Une première illustration préférée d'un vecteur constituant le vecteur d'ADN comprenant l'acide nucléique d'intérêt utilisé selon l'invention est le vecteur pCUL-lacZ :kan dont le procédé de construction est décrit dans les exemples.

Une seconde illustration préférée d'un vecteur constituant le vecteur d'ADN comprenant l'acide nucléique d'intérêt utilisé selon l'invention est le vecteur pGT-rev1 dont le procédé de construction est décrit dans les exemples.

On peut aussi avoir recours à des vecteurs capables d'inclure de grandes séquences d'insertion. Dans ce mode de réalisation particulier, on utilisera de préférence des vecteurs de bactériophage, tels que les vecteurs de bactériophage P1, comme le vecteur p158, ou encore le vecteur p158/neo8 décrits par Sternberg (1992, 1994).

Les vecteurs bactériens préférés selon l'invention sont par exemple les vecteurs pBR322(ATCC37017) ou encore des vecteurs tels que pAA223-3 (Pharmacia, Uppsala, Suède), et pGEM1 (Promega Biotech, Madison, WI, ETATS-UNIS).

On peut encore citer d'autres vecteurs commercialisés tels que les vecteurs pQE70, pQE60, pQE9 (Qiagen), psiX174, pBluescript SA, pNH8A, pNH16A, pNH18A, pNH46A, pWLNEO, pSV2CAT, pOG44, pXTI, pSG(Stratagene).

Il peut s'agir aussi du vecteur recombinant PXP1décrit par Nordeen SK et al. (1988).

Il peut encore s'agir de vecteurs adénoviraux tels que l'adénovirus humain de type 2 ou 5.

Un vecteur recombinant selon l'invention peut aussi être un vecteur rétroviral ou encore un vecteur adéno-associé (AAV). De tels vecteurs adéno-associés sont par exemple décrits par Flotte et al. (1992), Samulski et al. (1989), ou encore McLaughlin BA et al. (1996).

Selon un autre mode de réalisation du polynucléotide comprenant l'acide nucléique d'intérêt, le vecteur d'ADN est un ADN linéaire double brin.

De manière surprenante, on a montré que le procédé selon l'invention permettait l'insertion ciblée d'acides nucléiques de grande taille, supérieure à 1,5 kilobases. Cette caractéristique du procédé de l'invention est particulièrement avantageuse, car elle permet notamment l'insertion ciblée de séquences génomiques de gènes entiers, avec toutes les séquences fonctionnelles présentes dans les introns des gènes, qui ne sont pas retrouvées par exemple dans l'ADNc correspondant.

De manière générale, l'acide nucléique d'intérêt à insérer dans le génome de la cellule procaryote ou eucaryote inclus dans le vecteur d'ADN comprend au moins, respectivement à son extrémité 5' et à son extrémité 3', des séquences nucléotidiques ayant un haut degré d'identité, de préférence supérieur à 99,5%, et encore mieux supérieur à 99,6%, 99,7%, 99,8%, 99,9% d'identité, sans lacune ou « gap », avec les séquences correspondantes de l'ADN cible contenu dans le chromosome. De préférence, ces séquences localisées respectivement aux extrémités 5' et 3' de l'acide nucléique d'intérêt sont identiques aux séquences des extrémités respectivement 5' et 3' de la séquence cible visée qui est présente dans le chromosome, afin d'accroître encore la fréquence des évènements de recombinaison homologue. Les séquences flanquantes localisées respectivement aux extrémités 5' et 3' de l'acide nucléique d'intérêt inclus dans le vecteur utilisé pour la transfection comprennent au moins 100 paires de base, de préférence au moins 300 paires de base successives identiques aux séquences cibles correspondantes dans le chromosome. Plus la taille des séquences flanquantes 5' et 3' est grande et plus la probabilité d'obtention d'une haute fréquence de recombinaison homologue est élevée. De manière générale, on préfère des séquences flanquantes ayant de 300 à 1500 paires de bases identiques avec les séquences cibles correspondantes natives dans le chromosome.

Des séquences flanquantes homologues d'une longueur supérieure à 1500 paires de bases peuvent aussi être utilisées.

Selon un mode de réalisation tout à fait préféré du procédé, l'acide nucléique d'intérêt à insérer dans le génome, _qui est inclus dans le vecteur d'ADN, comprend une séquence marqueur de sélection. Préférentiellement, la séquence nucléotidique marqueur de sélection consiste en un gène marqueur de sélection qui s'exprime dans la cellule hôte procaryote ou eucaryote après l'insertion ciblée, par recombinaison homologue, dudit acide nucléique d'intérêt à l'endroit choisi du génome. Avantageusement, le gène marqueur de sélection est choisi parmi :
a) les gènes marqueurs de sélection fonctionnels chez *E. coli,* tels que les gènes de résistance à l'ampiciline, la tétracycline, la kanamycine, le chloramphénicol ;
b) les gènes marqueurs fonctionnels dans les cellules de mammifères, tels que les gènes de résistance à la néomycine ou à la zéocine.
c) les gènes marqueurs codant une protéine fluorescente telle qu'une GFP (« Green Fluorescent Protein ») ou YFP («-Yellow Fluorescent Protein »).

Pour choisir un gène marqueur de sélection approprié, l'homme du métier se référera avantageusement à l'ouvrage de Sambrook et al. (2001).

Le gène marqueur de sélection inclus dans l'acide nucléique d'intérêt permet ainsi aisément de réaliser l'étape c) de sélection du procédé selon l'invention.

En effet, la séquence marqueur de sélection incluse dans l'acide nucléique d'intérêt rend aisée la sélection des clones de cellules procaryotes ou eucaryotes initialement transfectées avec le vecteur d'ADN comprenant ledit acide nucléique d'intérêt, lesquelles cellules ont intégré, par recombinaison homologue, ledit acide nucléique d'intérêt, dans la localisation choisie de leur génome. L'événement de recombinaison homologue non réciproque du vecteur vers le chromosome peut ainsi être détecté par l'observation du phénotype des cellules procaryotes ou eucaryotes recombinées, ledit phénotype étant conféré par l'expression du gène marqueur, par exemple pour la production d'une protéine marqueur codée par le gène marqueur. La protéine marqueur peut être une protéine de résistance à un antibiotique ou encore une protéine fluorescente.

Selon un premier aspect préféré, l'acide nucléique d'intérêt qui est contenu dans-le vecteur comprend un cadre de lecture ouvert codant une protéine d'intérêt thérapeutique. La protéine d'intérêt thérapeutique peut être de toute nature. Il peut s'agir par exemple d'une protéine choisie parmi les cytokines, les hormones ou encore les facteurs de croissance.

Parmi les cytokines, on peut citer notamment les Interleukines, tels que l'IL-1, l'IL-2, l'IL-3, l'IL-4, l'IL-5, l'IL6, l'IL-10 ou l'IL-12, ou encore d'autres facteurs cytokiniques tels que MG-CSF.

Parmi les hormones, on peut citer notamment la LHRH. Parmi les facteurs de croissance, on peut citer notamment l'hormone de croissance humaine.

La protéine d'intérêt thérapeutique peut également être une protéine ou un peptide antigénique susceptible, lorsqu'il est présenté aux cellules du système immunitaire, d'induire la production d'anticorps à l'encontre d'un antigène, par exemple un antigène dérivé d'une bactérie ou d'un virus pathogène, ou encore induire la production de lymphocytes T-cytotoxiques spécifiques d'un antigène dérivé d'un organisme pathogène, tel qu'un rétrovirus comme le VIH-1 ou le VIH-2 ou le virus de l'hépatite virale, ou encore de lymphocytes T-cytotoxiques spécifiques d'antigènes tumoraux.

De préférence, selon ce premier aspect préféré de l'acide nucléique d'intérêt, le cadre de lecture ouvert code pour une protéine d'intérêt thérapeutique dont une surexpression est recherchée dans la cellule hôte.

Selon un second aspect préféré, l'acide nucléique d'intérêt comprend un cadre de lecture ouvert interrompu par une séquence nucléotidique hétérologue. Ce second aspect préféré de l'invention est mis en oeuvre principalement lorsque l'insertion ciblée de l'acide nucléique d'intérêt vise à remplacer, dans le chromosome cellulaire, au moins une partie de la séquence d'un gène par ledit acide nucléique d'intérêt, afin d'interrompre la séquence sauvage native dudit gène dans le chromosome et bloquer ainsi son expression. Un tel acide nucléique comprend au moins, de l'extrémité 5' vers l'extrémité 3', (i) une première séquence nucléotidique identique à une séquence du gène cible présente dans le chromosome, (ii) la séquence nucléotidique hétérologue et (iii) une seconde séquence nucléotidique identique à une seconde séquence du gène cible dans le chromosome. Les séquences nucléotidiques (i) et (ii), du fait qu'elles sont identiques à des séquences correspondantes dans le chromosome, permettent de cibler l'insertion de l'acide nucléique d'intérêt dans ledit gène.

Selon l'invention, la séquence nucléotidique hétérologue (ii) consiste en une séquence nucléotidique qui n'est pas naturellement présente dans l'ADN cible visé.

On utilisera en particulier ce second aspect préféré de l'acide nucléique d'intérêt de l'invention pour transfecter des cellules souches embryonnaires de mammifères non humains, dans un procédé de production d'animaux transgéniques dans lesquels le gène cible est interrompu et bloqué dans son expression (animaux knock-out).

Selon ce second aspect préféré, la séquence nucléotidique hétérologue (ii) peut être le gène marqueur défini précédemment, tel qu'un gène codant pour une protéine de résistance aux antibiotiques ou encore une toute autre protéine détectable, telle qu'une protéine fluorescente comme la GFP(« Green fluorescent protein ») ou encore YFP (« Yellow fluorescent protein »), bien connues de l'homme du métier.

Selon un troisième aspect préféré, l'acide nucléique d'intérêt code pour un ARN antisens. On mettra en oeuvre ce troisième aspect préféré de l'acide nucléique d'intérêt lorsque l'objectif recherché est d'inhiber l'expression d'une protéine codée par un gène cible, l'ARN antisens ainsi produit hybridant spécifiquement avec l'ARN messager constituant le produit de transcription du gène dont l'inhibition de l'expression est recherchée.

Selon un quatrième aspect préféré, le procédé selon l'invention est utilisé pour introduire une ou plusieurs mutations dans une séquence génomique d'une cellule procaryote ou eucaryote, par exemple une ou plusieurs mutations ponctuelles correspondant chacune à la substitution d'une base dans la séquence génomique ciblée initiale de la cellule procaryote ou eucaryote. Selon ce quatrième aspect préféré, l'acide nucléique d'intérêt inclus dans le vecteur d'ADN possède une séquence nucléotidique identique à celle de la séquence génomique ciblée, à l'exception de la ou des bases substituées.

De préférence, selon ce quatrième aspect préféré, les bases hétérologues par rapport à la séquence cible du génome cellulaire, qui sont incluses dans l'acide nucléique d'intérêt, sont avantageusement localisées à proximité de la séquence marqueur de sélection également incluse dans l'acide nucléique d'intérêt.

De manière tout à fait préférée, l'acide nucléique d'intérêt comprend, selon ce quatrième mode de réalisation, de 1 à 10 bases substituées, distinctes des bases correspondantes de l'ADN cible visé.

Ce quatrième mode de réalisation préféré est appliqué notamment pour corriger ou au contraire introduire des mutations spécifiques, de manière ciblée, dans des localisations prédéterminées du génome cellulaire, par exemple dans des procédés d'obtention d'animaux transgéniques de type « knock-in ».

Préférentiellement, l'acide nucléique d'intérêt inclus dans le vecteur d'ADN comprend de plus une séquence nucléotidique à fonction de promoteur, fonctionnelle dans la cellule hôte procaryote ou eucaryote choisie, sous le contrôle de laquelle est placé le cadre de lecture ouvert ou la séquence codant l'ARN antisens. Le type de promoteur sera choisi parmi les promoteurs connus, en fonction du type de cellules hôtes choisies, procaryotes ou eucaryotes.

A titre d'exemples, les promoteurs bactériens pourront être les promoteurs Lacl, LacZ, les promoteurs de l'ARN polymérase du bactériophage T3 ou T7, les promoteurs PR, ou PL du phage lambda.

Les promoteurs pour cellules eucaryotes comprendront le promoteur de la thymidine kinase du virus HSV ou encore le promoteur de la métallothionéine-L de souris.

De manière générale, pour le choix d'un promoteur adapté, l'homme du métier pourra avantageusement se référer à l'ouvrage de Sambrook et al. (1989) ou encore aux techniques décrites par Fuller et al. (1996).

Selon encore un autre mode de réalisation préféré de l'invention, le vecteur d'ADN comprenant l'acide nucléique d'intérêt qui est utilisé pour transfecter les cellules eucaryotes ou procaryotes, comprend une séquence nucléotidique marqueur, notamment un gène marqueur, par exemple une séquence nucléotidique codant une protéine marqueur, ladite séquence nucléotidique étant localisée, dans ledit vecteur, en dehors de la séquence nucléotidique de l'acide nucléique d'intérêt. Ce gène marqueur permet aisément la réalisation de l'étape d) de sélection du procédé selon l'invention. Selon ce mode de réalisation préféré de l'invention, l'expression de la protéine marqueur codée par la séquence nucléotidique localisée en dehors de la séquence de l'acide nucléique d'intérêt permet de sélectionner les cellules hôtes procaryotes ou eucaryotes qui ont été efficacement transfectées par ledit vecteur d'ADN, par exemple un plasmide ou tout autre vecteur d'ADN, mais pour lesquels l'événement de recombinaison homologue n'a pas eu lieu avec élimination des séquences nucléotidiques du vecteur, autres que celle de l'acide nucléique d'intérêt, par exemple avec élimination des séquences du vecteur autres que celles de l'acide nucléique d'intérêt à insérer.

L'introduction du vecteur d'ADN comprenant l'acide nucléique d'intérêt selon l'invention dans une cellule est réalisée *in vitro,* selon les techniques bien connues de l'homme du métier pour transformer ou transfecter des cellules, soit en culture primaire, soit sous la forme de lignées cellulaires.

Pour introduire les vecteurs dans une cellule hôte, l'homme du métier pourra avantageusement se référer à différentes techniques, comme la technique de précipitation au phosphate de calcium (Graham et al., 1973 ; Chen et al., 1987), le DEAE Dextran (Gopal, 1985), l'électroporation (Tur-Kaspa, 1896), la microinjection directe (Harland et al., 1985), ou encore les liposomes chargés en ADN (Nicolau et al., 1982, Fraley et al., 1979).

Le procédé selon l'invention trouve une application préférée pour l'insertion ciblée de l'acide nucléique d'intérêt dans une cellule bactérienne ou dans une cellule de mammifère, humaine ou non-humaine.

Lorsque le procédé selon l'invention est mis en oeuvre pour l'insertion ciblée d'un acide nucléique d'intérêt dans le génome d'une cellule de mammifère, en particulier dans une localisation prédéterminée du génome d'une cellule humaine, il s'intègre en tant qu'étape particulière de réalisation d'un procédé de thérapie génique des cellules somatiques.

La thérapie génique consiste à corriger une déficience ou une anomalie (mutation, expression aberrante, etc) ou à assurer l'expression d'une protéine d'intérêt thérapeutique par introduction d'une information génétique dans la cellule ou l'organe affecté. Cette information génétique peut être introduite soit *ex vivo* dans une cellule extraite de l'organe, la cellule modifiée étant alors réintroduite dans l'organisme, soit directement *in vivo* dans le tissu approprié. Dans ce second cas, différentes techniques existent, parmi lesquelles des techniques diverses de transfection impliquant des complexes d'ADN et de DEAE-dextran (Pagano et al., 1967), d'ADN et de protéines nucléaires (Kaneda et al, 1989), d'ADN et de lipides (Felgner et al, 1987), l'emploi de liposomes (Fraley et al., 1980), etc. Plus récemment, l'emploi de virus comme vecteurs pour le transfert de gènes est apparu comme une alternative prometteuse à ces techniques physiques de transfection. A cet égard, différents virus ont été testés pour leur capacité à infecter certaines populations cellulaires. En particulier, les rétrovirus (RSV, HMS, MMS, etc), le virus HSV, les virus adéno-associés, et les adénovirus.

Selon encore un autre aspect préféré, les cellules transférées à l'étape b) du procédé de l'invention consistent en des cellules bactériennes, telles que *E. coli.*

Selon un autre aspect préféré, les cellules transfectées à l'étape b) du procédé de l'invention consistent en des cellules de mammifères non humains, telles que des cellules de souris ou de lapins, y compris les cellules souches embryonnaires de la lignée ES, ou encore des cellules de rats, de cobayes, de chiens ou de singes.

Selon un dernier aspect préféré de l'invention, les cellules transfectées à l'étape b) du procédé consistent en des cellules humaines, comme par exemple des cellules épithéliales, des cellules musculaires, des monocytes/macrophages ou encore des lymphocytes.

La présente invention est en outre illustrée, sans pour autant être limitée, à la figure unique et aux exemples suivants.

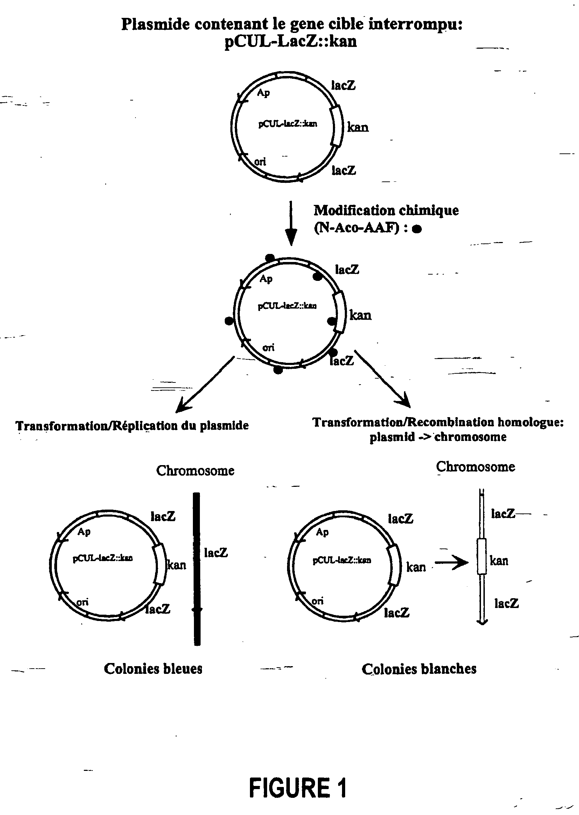

La figure 1 illustre le schéma expérimental d'insertion d'un acide nucléique d'intérêt contenant le gène LacZ interrompu par un gène marqueur de résistance à la kanamycvine dans le génome d'une cellule de *Escherichia coli.*

La figure 2 illustre le schéma expérimental de construction d'un vecteur d'ADN pour l'insertion ciblée d'un acide nucléique d'intérêt dans des cellules humaines, le vecteur pGT-Rev1. Dans le vecteur pGT-Rev1, l'acide nucléique d'intérêt est l'acide nucléique Rev1 codant pour une polymérase humaine, dont la séquence est interrompue par un gène marqueur, ici le gène de résistance à la Zéocine ou le gène de résistance à la Blasticidine.

La Figure 3 illustre le schéma de transfection *in vitro* de cellules humaines avec le vecteur pGT-Rev1, en vue d'insérer dans le chromosome une copie non fonctionnelle du gène Rev1, interrompue par le gène marqueur.

### EXEMPLES

### EXEMPLE 1 : Construction du vecteur pCUL-lacZ ::kan

Le plasmide pCUL-LacZ ::kan a été construit à partir d'un dérivé du vecteur bien connu pUC8 (décrit par YANNISCH-PERRON et al., 1985) dans lequel l'origine de réplication ainsi que le gène LacZ' ont été retournés (vecteur pCUL-, cf Schumacher et al. 1998). Le gène LacZ a été obtenu par traitement d'un dérivé du plasmide pBR329 contenant le gène LacZ à l'aide des endonucléases de restriction EcoRI « fill-in » et Fspl. Le fragment de 3000 bp ainsi obtenu a ensuite été cloné dans les sites Sapl (filled in) in Fspl du vecteur pCUL-. Le plasmide résultant (pCUL-LacZ) contient l'origine de réplication Co1EI, le marqueur de résistance à l'ampicilline ainsi que le gène lacZ en entier. Le gène de résistance à la Kanamycine, cloné par PCR à partir du vecteur pUC4k (Pharmacia) a été ensuite inséré au site EcoRV du plasmide pCUL-LacZ pour donner naissance au plasmide pCUL-LacZ ::kan utilisé dans la suite des expériences.

### EXEMPLE 2 : Insertion ciblée, par recombinaison homologue, d'un acide nucléique d'intérêt dans un génome cellulaire procaryote

### A. Matériel et Méthodes.

### A.1 Traitement du vecteur par le N-AcO-AAF.

### Mélange réactionnel :

· ADN plasmidique : concentration finale de 0,5µg/µl dans du tampon citrate 2 x 10⁻³ M, pH7.
· N-AcO-AAF : concentration finale de 400µg/ml dans de l'éthanol.

On ajoute 60 µg d'ADN dans 114 µl de tampon citrate 2 x 10⁻³ M pH7, additionnée de 3 µl d'éthanol à 100% ;
On préchauffe la solution à 37°C ;
puis on ajoute 3 µl de N-AcO-AAF (400 µg/ml) marqué au tritium (³H).

Temps de réaction : 4, 8, 20 minutes.

On prélève 40 µl aux différents temps choisis. On arrête la réaction par une étape de précipitation de l'ADN avec 3 volumes d'éthanol/acétate de sodium à 0,16 M suivi de trois autres étapes de précipitation dans les mêmes conditions.

On détermine le pourcentage de bases modifiées en mesurant le nombre de moles de plasmide par spectrophotométrie UV et le nombre de moles d'AAF fixé, par comptage de la radioactivité β.

Le résultat est exprimé en pourcentage de bases modifié. Le temps de réaction est adapté en fonction de la taille du plasmide et du pourcentage de bases modifié souhaité.

### A.2. Réalisation de la transfection des cellules de E.coli

Les cellules de *E.coli* sont transformées selon le procédé conventionnel d'électroporation en utilisant les conditions préconisées par les constructeurs des appareils prévu pour cet effet (Gene Pulser de Biorad par exemple).

### A.3. Sélection des clones cellulaires recombinants

Dans l'exemple, les clones transformants sont sélectionnés sur milieu LB-agar contenant de la Kanamycine (20µg/ml) et l'absence d'activité β-galactosidase (se traduisant par des colonies blanches au lieu de bleues), phénotype caractéristique d'un événement de recombinaison non réciproque est visualisé grâce à la présence de X-gal (5-bromo-4-chloro-3-indolyl-β-D-gafactopyranoside) et d'IPTG (isopropyl-β-D-thiogalactopyranoside) dans les boîtes.

### B. RESULTATS

Les résultats sont représentés dans le tableau 1 ci-après.

**TABLEAU 1**

| Nombre de lésions par plasmide | Fréquence de recombinaison : colonies blanches/colonies totales |
|---|---|
| OAAF | <0.0096^{a} |
| | 10426/0^{b} |
| 10AA2F | 0.056 |
| | 17819/10 |
| 20AAF | 0.17 |
| | 9692/17 |
| 33AAF | 0.32 |
| | 2301975 |
| 43AAF | 1.41 |
| | 16554/237 |
| 67AAF | 6.07 |
| | 1934/125 |

| | |
|---|---|
| ^{a} fréquence de recombinaison homologue ^{b} nombre total de colonies/nombre de colonies recombinées. | |

Les résultats présentés dans le Tableau 1 ci-dessus montrent clairement que la fréquence de recombinaison homologue ciblée augmente en fonction du nombre de lésions produites par la modification chimique du plasmide (tableau 1). Ainsi, dans une souche sauvage pour les systèmes de recombinaison, alors que les évènements spontanés de recombinaison homologue représentent moins de 0,03% des évènements de transformation, la présence de 67 lésions AAF sur ce plasmide conduit à l'obtention de plus de 6% de molécules recombinantes ciblées, soit une augmentation de plus de 1700 fois de la fréquence de recombinaison.

De plus, on a mis en évidence qu'avec le procédé de l'invention, le mécanisme conduisant au ciblage du gène est un mécanisme de recombinaison homologue non réciproque au cours duquel la molécule plasmidique est perdue. Ceci permet de réaliser un ciblage de gène très efficace en une seule étape. Chez *E. coli*, ce procédé a été utilisé pour construire plus de dix souches dans lesquelles différents gènes ont été interrompus.

Ainsi, par exemple, ont été construites des souches dans lesquelles certains gènes impliqués dans la recombinaison (recF, recG, dinG), ont été interrompus par des gènes codant pour la résistance à des antibiotiques comme la tétracycline ou le chloramphénicol. Ce ciblage de gène a été réalisé dans différents fonds génétiques comme par exemple dans des souches dont le système de réparation par excision (uvrABC dépendant) était inactivé.

### EXEMPLE 2 : Insertion ciblée, par recombinaison homologue, d'un acide nucléique d'intérêt dans un génome cellulaire eucaryote humain

### A. Construction du vecteur pGT-Rev1

On a construit le vecteur pGT-Rev1 destiné à être utilisé dans le procédé selon l'invention, en vue d'insérer une copie non fonctionnelle du gène Rev1 dans le-génome d'une cellule humaine, en remplacement de la copie du gène Rev1 natif.

Le vecteur pGT-Rev1 comprend une origine de réplication fonctionnelle chez *E. coli,* ici l'origine ColE1, ce qui permet, après transfection de cellules de *E. coli* avec ce vecteur, l'obtention de quantités suffisantes du vecteur pour transfecter ensuite les cellules humaines cibles, en vue d'insérer le gène Rev1 interrompu par le gène marqueur, à la place du gène Rev1 natif. Pour vérifier le succès de la transfection chez *E. coli,* le vecteur PGT-Rev1 comprend un gène marqueur de sélection qui est fonctionnel chez les bactéries, le gène de résistance à l'ampicilline.

Le vecteur pGT-Rev1 comprend une origine de réplication fonctionnelle dans le cellules humaines, ici l'origine de réplication oriP du virus d'Epstein-Barr, qui permet au vecteur de se répliquer un nombre limité de fois dans les cellules humaines. Pour vérifier le succès de la transfection dans les cellules humaines, le vecteur PGT-REV1 comprend un gène marqueur de transfection qui est fonctionnel chez l'homme, le gène eGFP codant la « Green Fluorescent Protein ».

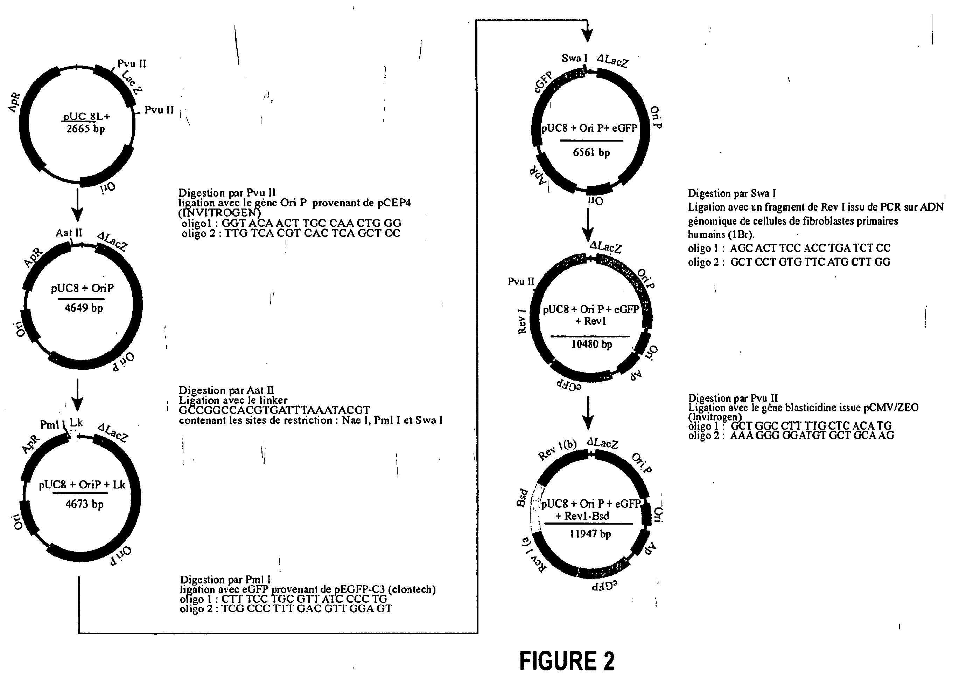

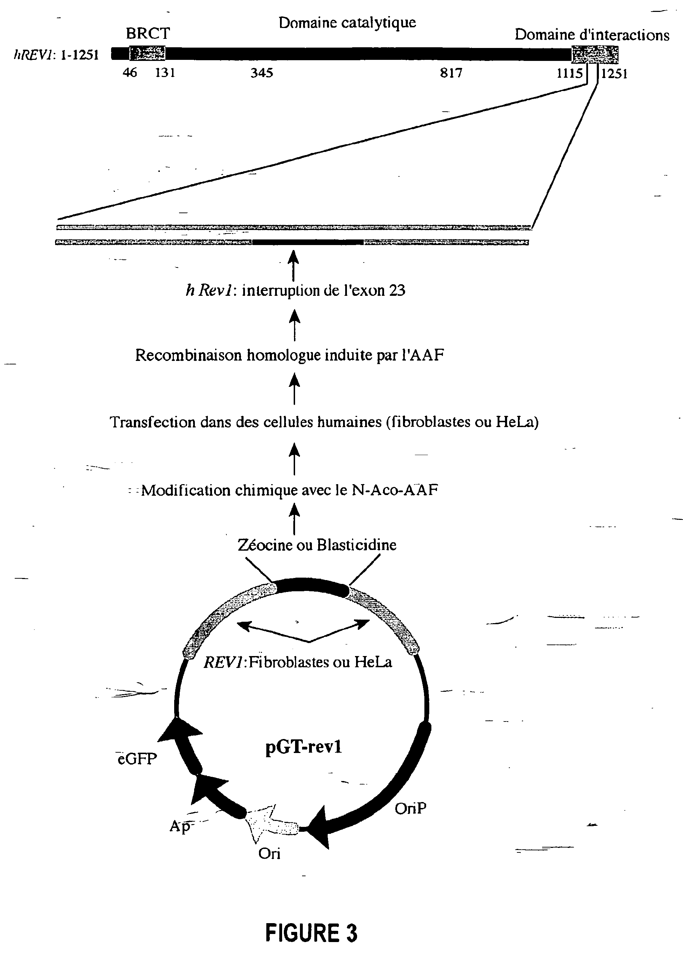

Les différentes étapes du protocole de construction du vecteur pGT est décrit sur le schéma de la figure 2. Il apparaîtra clairement à l'homme du métier que l'acide nucléique d'intérêt est inséré dans le vecteur pGT décrit dans la figure 2, pour obtenir le vecteur d'ADN utilisé dans le procédé selon l'invention. Notamment, la figure 3 illustre le vecteur PGT-Rev1, qui est le vecteur pGT dans lequel on a inséré, en tant qu'acide nucléique d'intérêt, le gène humain Rev1 dont la séquence nucléotidique a été interrompue par un gène marqueur.

Comme illustré sur la Figure 2, pour réaliser le vecteur pGT, on a utilisé les oligonucléotides suivants :
a) Etape 1 :
   oligo 1 : GGTACAACTTGCCAACTGGG (SEQ ID N°1) ;
   oligo 2 : TTGTCACGTCACTCAGCTCC (SEQ ID N°2) ;
b) Etape 2 :
   GCCGGCCACGTGATITAAATACGT (SEQ ID N°3) ;
c) Etape 3 :
   oligo 1 : CTTTCCTGCGTTATCCCCTG (SEQ ID N°4) ;
   oligo 2 : TCGCCCTTTGACGTTGGAGT (SEQ ID N°5) ;
d) Etape 4 :
   oligo 1 : AGCACTTCCACCTGATCTCC (SEQ ID N°6) ;
   oligo 2 : GCTCCTGTGTTCTTCATGCTTGG (SEQ ID N°7) ;
e) Etape 5 :
   oligo 1 : GCTGGCCTTTTGCTCACATG (SEQ ID N°8) ;
   oligo 2 : AAAGGGGGATGTGCTGCAAG (SEQ ID N°9).

En utilisant le principe développé chez *E. coli,* on a adapté l'outil décrit dans les exemples précédents, pour la transfection des cellules humaines en culture.

Pour cela on a développé un vecteur dont les caractéristiques sont les suivantes : i) il est capable de se répliquer dans la bactérie de telle sorte que l'on puisse produire suffisamment de plasmide pour le modifier chimiquement ; ii) il est également capable de se répliquer dans les cellules humaines afin de générer des extrémités recombinogènes par blocage de la réplication au niveau des lésions. Par conséquent, l'ossature de ce vecteur contient les éléments suivants :
- Origine de réplication fonctionnant chez *E. coli* (ColE1, grand nombre de copies).
- Marqueur de sélection bactérien : résistance à l'ampicilline
- Origine de réplication du virus Epstein-Barr (oriP) lui permettant de se répliquer un nombre limité de fois dans des cellules-humaines.
- Marqueur de transfection: Green Fluorescent Protein (eGFP).

### B. Ciblage du gène REV1 humain

Le produit du gène *REV1* fait partie d'une nouvelle classe de polymérases spécialisées (dont font partie également les ADN polymérases êta (Polη), iota (Polτ) et kappa (Polκ) qui ont pour caractéristique la structure particulière de leur site actif leur permettant de tolérer et de répliquer l'ADN contenant des lésions ou des distorsions. Il a été montré récemment dans notre laboratoire, que la protéine codée par le gène *REV1* et l'ADN polymérase êta interagissent et la région de cette interaction sur la protéine Rev1 a été mappée. Polη joue un rôle primordial dans la cellule car elle est capable de franchir très efficacement et sans erreur un dimère de pyrimidine T-T de type cyclobutane (lésion majoritairement formée après irradiation des cellules aux rayonnements ultraviolets). L'absence de cette polymérase est à l'origine de la maladie *Xeroderma pigmentosum* variant (XPV) et entraine l'hypermutabilité des cellules après irradiation aux UV et l'apparition de cancers cutanés

Si l'interaction entre Rev1 et Polη est avérée, son rôle physiologique reste inconnu et posséder un mutant de Rev1 n'interagissant plus avec Polη permettrait bien évidemment de comprendre l'importance de cette interaction.

Le demandeur a ciblé le site d'interaction porté par Rev1, en utilisant la stratégie suivante :
a) Amplification par PCR du fragment chromosomique contenant l'exon 23 du gène *REV1,* issu soit de cellules 1BR (fibroblastes primaires) soit de cellules HeLa (cellules humaines transformées) ; séquençage du produit PCR et clonage dans les plasmides « ad hoc » de façon à disposer des deux allèles sur plasmide;
b) Interruption de l'exon 23 de chaque allèle cloné par un gène codant pour la résistance à un antibiotique (blasticidine ou zéocine) ;
c) Modification des plasmides ainsi obtenus par le N-Aco-AAF ; et
d) Transfection dans les cellules humaines (1Br ou Hela) en culture et selection pour la résistance à l'antibiotique de choix.

### RESULTATS

Une série d'expériences a été réalisée : les plasmides contenant le fragment du gène *REV1* issu de fibroblastes primaires (1BR) ont été produits en quantité, et traités avec le N-Aco-AAF. Les plasmides traités ont ensuite été utilisés pour transfecter des cellules de fibroblastes humains en culture primaire.

L'analyse de la fluorescence induite par la GFP montre que de nombreuses cellules ont été transfectées.

L'application de la sélection par les antibiotiques a permit d'obtenir des clones recombinants.

### REFERENCES

Cappecchi, 1989, Science, vol. 244 : 1288
Chen et al., 1987, Mol. Cell. Biol., 7 : 2745-2752.
Fraley et al., J.Biol.Chem. 255 (1980) 10431
Flotte et al., 1992, Am. J. Respir. Cell Mol. Biol., 7 : 349-356.
Fuchs R.P.P. J., 1984, Mol. Biol., 177(1): 173:180**.**
Felgner et al., 1987, Proc. Natl. Acad. Sci. USA, 84 : 7413
Fuller S.A. et al., 1996, Immunology in Current Protocols in Molecular Biology, Ausubel et al.
Graham et al., 1973, Virology, 52 : 456-457.
Gopal, 1985, Mol. Cell. Biol., 5: 1188-1190.
Harland et al., 1985, J. Cell. Biol., 101 : 1094-1095.
Hinds et al., 1999, Microbiology, 154, 519-527.
Kaneda et al., 1989, Science 243 : 375
Lefèvre et al., 1978, Biochemistry, vol.17 : 2561-2567.
Luisi-De-Luca C, 1984, Proc. Natl. Acad. Sci. USA, vol.81 :2831-2835
Maher et al., 1990, Environmental Science Research, 149-156
McLaughlin BA et al., 1996, Am. J. Hum. Genet., 59 : 561-569.
Nicolau C. et al., 1987, Methods Enzymol., 149:157-76.
Nordeen SK et al., 1988, BioTechniques, 6 : 454-457
Pagano et al., 1967, J.Virol. 1 : 891
Reid et al., 1991, Molecular and Cellular Biology, vol. 11: 2769
Samulski et al., 1989, J. Virol., 63 : 3822-3828.
Sambrook, J. Fritsch, E. F., and T. Maniatis. 1989. Molecular cloning: a laboratory manual. 2ed. Cold Spring Harbor Laboratory, Cold spring Harbor, New York.
Sternberg N.L., 1992, Trends Genet., 8 : 1-16.
Sternberg N.L., 1994, Mamm. Genome, 5 : 397-404.
Schmid S E, 1982, Proc. Natl. Acad. Sci. USA, vol.79:4133(4137.
Sambrook et Russel, Molecular Cloning, 2001, 3ème édition, CSHL Press.
**Schumacher et al**., 1998 **[Référence à compléter S.V.P.].**
Tur-Kaspa et al, 1986, Mol. Cell. Biol., 6 : 716-718.
Yannisch-Perron C. et al., 1985, Gene, 33:103-119.

## Revendications

1. Procédé pour insérer *in vitro* un acide nucléique d'intérêt inclus initialement dans un vecteur d'ADN, au sein d'une séquence nucléotidique prédéterminée présente dans un chromosome contenu dans une cellule procaryote ou eucaryote, **caractérisé en ce qu'**il comprend les étapes de :
a) mettre en contact le vecteur d'ADN comprenant l'acide nucléique d'intérêt, et qui se réplique dans ladite cellule procaryote ou eucaryote, avec un agent mutagène bloquant la réplication de l'ADN dans la cellule ;
b) transfecter des cellules procaryotes ou eucaryotes avec le vecteur d'ADN tel qu'obtenu à la fin de l'étape a) ;
c) sélectionner les cellules procaryotes ou eucaryotes pour lesquelles l'acide nucléique d'intérêt a été intégré dans la séquence nucléotidique prédéterminée.

2. Procédé selon la revendication 1, **caractérisé en ce qu'**il comprend en outre l'étape suivante :
d) sélectionner parmi les cellules procaryotes ou eucaryotes sélectionnées à l'étape c), les cellules dans lesquelles les séquences du vecteur d'ADN, autres que celle de l'acide nucléique d'intérêt, ont été éliminées.

3. Procédé selon la revendication 1, **caractérisé en ce que** l'agent mutagène est choisi parmi le N-acétoxy-2-acétylaminofluorène (N-AcO-AAF), un agent alkylant, le benzo(a) pyrene-diol-époxyde (BPDE) ou encore un rayonnement UV.

4. Procédé selon la revendication 3, **caractérisé en ce que** l'agent mutagène est le N-acétoxy-2-acétylaminofluorène (N-AcO-AAF).

5. Procédé selon la revendication 4, **caractérisé en ce qu'**à l'étape a), le N-AcO-AAF est mis en contact avec le vecteur d'ADN comprenant l'acide nucléique d'intérêt, à une concentration adaptée à la fixation d'au moins 10 molécules de N-AcO-AAF par molécule du polynucléotide.

6. Procédé selon la revendication 5, **caractérisé en ce que** la concentration de N-AcO-AAF est adaptée à la fixation d'au moins 50 molécules de N-AcO-AAF par molécule du polynucléotide.

7. Procédé selon l'une des revendications 1 à 6, **caractérisé en ce que** l'acide nucléique d'intérêt à insérer dans le génome de la cellule procaryote ou eucaryote, qui est inclus initialement dans ledit vecteur d'ADN, comprend respectivement, à son extrémité 5' et à son extrémité 3', des séquences ayant un haut degré d'identité avec les séquences correspondantes localisées- aux extrémités 5' et 3' de l'ADN cible contenu dans le chromosome.

8. Procédé selon la revendication 7, **caractérisé en ce que** les séquences localisées respectivement à l'extrémité 5' et à l'extrémité 3' de l'acide nucléique d'intérêt sont identiques aux extrémités respectivement 5' et 3' de l'ADN cible contenu dans le chromosome.

9. Procédé selon l'une des revendications 1 à 8, **caractérisé en ce que** l'acide nucléique d'intérêt inclus dans ledit vecteur d'ADN comprend une séquence nucléotidique marqueur de sélection.

10. Procédé selon l'une des revendications 1 à 9, **caractérisé en ce que** l'acide nucléique d'intérêt comprend un cadre de lecture ouvert codant une protéine d'intérêt thérapeutique.

11. Procédé selon l'une des revendications 1 à 9, **caractérisé en ce que** l'acide nucléique d'intérêt comprend un cadre de lecture ouvert interrompu par une séquence nucléotidique hétérologue.

12. Procédé selon l'une des revendications 1 à 10, **caractérisé en ce que** l'acide nucléique d'intérêt code pour un ARN antisens.

13. Procédé selon l'une des revendications 10 à 12, **caractérisé en ce que** l'acide nucléique d'intérêt comprend de plus une séquence nucléotidique à fonction de promoteur, fonctionnelle dans la cellule hôte procaryote ou eucaryote choisie, sous le contrôle de laquelle est placé le cadre de lecture ouvert ou la séquence codant l'ARN incluse dans ledit acide nucléique d'intérêt.

14. Procédé selon l'une des revendications 1 à 13, **caractérisé en ce que** le polynucléotide comprenant l'acide nucléique d'intérêt comprend une séquence nucléotidique marqueur localisée, dans ledit polynucléotide, en dehors de la séquence nucléotidique de l'acide nucléique d'intérêt.

15. Procédé selon l'une des revendications 1 à 14, **caractérisé en ce que** le vecteur d'ADN est un plasmide bactérien.

16. Procédé selon l'une des revendications 1 à 15, **caractérisé en ce que** le vecteur d'ADN est un plasmide fonctionnel dans des cellules bactériennes.

17. Procédé selon l'une des revendications 1 à 15, **caractérisé en ce que** le vecteur d'ADN est un plasmide fonctionnel dans des cellules humaines.

18. Procédé selon l'une des revendications 1 à 17, **caractérisé en ce que** le vecteur d'ADN est un ADN linéaire double brin.

19. Procédé selon l'une des revendications 1 à 18 **caractérisé en ce que** les cellules transfectées à l'étape b) consistent en des cellules bactériennes.

20. Procédé selon l'une des revendications 1 à 18, **caractérisé en ce que** les cellules transfectées à l'étape b) consistent en des cellules de mammifère non humain.

21. Procédé selon l'une des revendications **caractérisé en ce que** les cellules transfectées à l'étape b) consistent en des cellules humaines, à l'exception des cellules germinales humaines et des cellules souches embryonnaires humaines.

## Claims

1. Process for the *in vitro* insertion of a nucleic acid of interest, initially included in a DNA vector, into a predetermined nucleotide sequence present in a chromosome contained in a prokaryotic or eukaryotic cell, **characterized in that** it comprises the following steps:
a) bringing the DNA vector which comprises the nucleic acid of interest, and which replicates in said prokaryotic or eukaryotic cell, into contact with a mutagenic agent that blocks the replication of the DNA in the cell;
b) transfecting prokaryotic or eukaryotic cells with the DNA vector as obtained at the end of step a); and
c) selecting the prokaryotic or eukaryotic cells for which the nucleic acid of interest has been integrated into the predetermined nucleotide sequence.

2. Process according to Claim 1, **characterized in that** it also comprises the following step:
d) selecting, from the prokaryotic or eukaryotic cells selected in step c), the cells from which the sequences of the DNA vector other than the sequence of the nucleic acid of interest have been removed.

3. Process according to Claim 1, **characterized in that** the mutagenic agent is selected from N-acetoxy-2-acetylaminofluorene (N-AcO-AAF), an alkylating agent, benzo(a)pyrene diol epoxide (BPDE) and UV radiation.

4. Process according to Claim 3, **characterized in that** the mutagenic agent is N-acetoxy-2-acetylaminofluorene (N-AcO-AAF).

5. Process according to Claim 4, **characterized in that**, in step a), the N-AcO-AAF is brought into contact with the DNA vector comprising the nucleic acid of interest at a concentration suitable for fixing at least 10 molecules of N-AcO-AAF per molecule of polynucleotide.

6. Process according to Claim 5, **characterized in that** the concentration of N-AcO-AAF is suitable for fixing at least 50 molecules of N-AcO-AAF per molecule of polynucleotide.

7. Process according to one of Claims 1 to 6, **characterized in that** the nucleic acid of interest to be inserted into the genome of the prokaryotic or eukaryotic cell, which is initially included in said DNA vector, respectively comprises, at its 5' end and at its 3' end, sequences having a high degree of identity with the corresponding sequences located at the 5' and 3' ends of the target DNA contained in the chromosome.

8. Process according to Claim 7, **characterized in that** the sequences located respectively at the 5' end and at the 3' end of the nucleic acid of interest are identical to the respective 5' and 3' ends of the target DNA contained in the chromosome.

9. Process according to one of Claims 1 to 8, **characterized in that** the nucleic acid of interest included in said DNA vector comprises a selection marker nucleotide sequence.

10. Process according to one of Claims 1 to 9, **characterized in that** the nucleic acid of interest comprises an open reading frame coding for a protein of therapeutic interest.

11. Process according to one of Claims 1 to 9, **characterized in that** the nucleic acid of interest comprises an open reading frame interrupted by a heterologous nucleotide sequence.

12. Process according to one of Claims 1 to 10, **characterized in that** the nucleic acid of interest codes for an antisense RNA.

13. Process according to one of Claims 10 to 12, **characterized in that** the nucleic acid of interest further comprises a nucleotide sequence with a promoter function that is functional in the chosen prokaryotic or eukaryotic host cell, under the control of which is placed the open reading frame or the RNA coding sequence included in said nucleic acid of interest.

14. Process according to one of Claims 1 to 13, **characterized in that** the polynucleotide comprising the nucleic acid of interest comprises a marker nucleotide sequence that is located, in said polynucleotide, outside the nucleotide sequence of the nucleic acid of interest.

15. Process according to one of Claims 1 to 14, **characterized in that** the DNA vector is a bacterial plasmid.

16. Process according to one of Claims 1 to 15, **characterized in that** the DNA vector is a plasmid that is functional in bacterial cells.

17. Process according to one of Claims 1 to 15, **characterized in that** the DNA vector is a plasmid that is functional in human cells.

18. Process according to one of Claims 1 to 17, **characterized in that** the DNA vector is a double-stranded linear DNA.

19. Process according to one of Claims 1 to 18, **characterized in that** the cells transfected in step b) consist of bacterial cells.

20. Process according to one of Claims 1 to 18, **characterized in that** the cells transfected in step b) consist of non-human mammalian cells.

21. Process according to one of Claims 1 to 18, **characterized in that** the cells transfected in step b) consist of human cells with the exception of human germinal cells and human embryonic stem cells.

## Patentansprüche

1. Verfahren zur in vitro-Insertion einer Nucleinsäure von Interesse, die ursprünglich in einem DNA-Vektor enthalten ist, in eine vorbestimmte Nucleotidsequenz, die in einem Chromosom vorliegt, das in einer Prokaryoten- oder Eukaryotenzelle enthalten ist, **dadurch gekennzeichnet, dass** es die folgenden Stufen umfasst:
a) In-Kontakt-Bringen des DNA-Vektors, der die Nucleinsäure von Interesse umfasst und der sich in der Prokaryoten- oder Eukaryotenzelle repliziert, mit einem Mutagen, das die Replikation der DNA in der Zelle blockiert;
b) Transfizieren der Prokaryoten- oder Eukaryotenzellen mit dem DNA-Vektor, wie er am Ende der Stufe a) erhalten wurde;
c) Selektionieren der Prokaryoten- oder Eukaryotenzellen, für die die Nucleinsäure von Interesse in die vorbestimmte Nucleotidsequenz integriert wurde.

2. Verfahren nach Anspruch 1, **dadurch gekennzeichnet, dass** es außerdem die folgende Stufe umfasst:
d) Selektionieren unter den Prokaryoten- oder Eukaryotenzellen, die in Stufe c) selektioniert wurden, die Zellen, in denen die Sequenzen des DNA-Vektors, die andere als die der Nucleinsäure von Interesse sind, eliminiert wurden.

3. Verfahren nach Anspruch 1, **dadurch gekennzeichnet, dass** das Mutagen ausgewählt ist unter N-Acetoxy-2-acetylaminofluoren (N-AcO-AAF), einem Alkylierungsmittel, Benzo(a)pyrendiolepoxid (BPDE) und UV-Strahlung.

4. Verfahren nach Anspruch 3, **dadurch gekennzeichnet, dass** das Mutagen N-Acetoxy-2-acetylaminofluoren (N-AcO-AAF) ist.

5. Verfahren nach Anspruch 4, **dadurch gekennzeichnet, dass** in der Stufe a) das N-AcO-AAF mit dem DNA-Vektor, der die Nucleinsäure von Interesse umfasst, in einer Konzentration in Kontakt gebracht wird, die an die Fixierung von wenigstens 10 Molekülen N-AcO-AAF pro Molekül Polynucleotid angepasst ist.

6. Verfahren nach Anspruch 5, **dadurch gekennzeichnet, dass** die Konzentration an N-AcO-AAF an die Fixierung von wenigstens 50 Molekülen N-AcO-AAF pro Molekül Polynucleotid angepasst ist.

7. Verfahren nach einem der Ansprüche 1 bis 6, **dadurch gekennzeichnet, dass** die Nucleinsäure von Interesse zur Insertion in das Genom der Prokaryoten- oder Eukaryotenzelle, die ursprünglich in dem DNA-Vektor enthalten ist, an ihrem 5'-Ende bzw. ihrem 3'-Ende Sequenzen umfasst, die einen hohen Identitätsgrad mit den entsprechenden Sequenzen haben, die an dem 5'- und 3'-Ende der Target-DNA lokalisiert sind, die im Chromosom enthalten ist.

8. Verfahren nach Anspruch 7, **dadurch gekennzeichnet, dass** die Sequenzen, die am 5'-Ende bzw. am 3'-Ende der Nucleinsäure von Interesse lokalisiert sind, mit dem 5'- bzw. 3'-Ende der Target-DNA, die im Chromosom enthalten ist, identisch sind.

9. Verfahren nach einem der Ansprüche 1 bis 8, **dadurch gekennzeichnet, dass** die Nucleinsäure von Interesse, die in dem DNA-Vektor enthalten ist, eine Selektionsmarker-Nucleotidsequenz umfasst.

10. Verfahren nach einem der Ansprüche 1 bis 9, **dadurch gekennzeichnet, dass** die Nucleinsäure von Interesse ein offenes Leseraster umfasst, das für ein Protein von therapeutischem Interesse codiert.

11. Verfahren nach einem der Ansprüche 1 bis 9, **dadurch gekennzeichnet, dass** die Nucleinsäure von Interesse ein offenes Leseraster umfasst, das durch eine heterologe Nucleotidsequenz unterbrochen ist.

12. Verfahren nach einem der Ansprüche 1 bis 10, **dadurch gekennzeichnet, dass** die Nucleinsäure von Interesse für eine Antisense-RNA codiert.

13. Verfahren nach einem der Ansprüche 10 bis 12, **dadurch gekennzeichnet, dass** die Nucleinsäure von Interesse darüber hinaus eine Nucleotidsequenz mit Promotorfunktion, die in der gewählten Prokaryoten- oder Eukaryoten-Wirtszelle funktionell ist, umfasst, unter deren Kontrolle das offene Leseraster oder die Sequenz, die für RNA codiert, die in der Nucleinsäure von Interesse enthalten ist, gestellt ist.

14. Verfahren nach einem der Ansprüche 1 bis 13, **dadurch gekennzeichnet, dass** das Polynucleotid, das die Nucleinsäure von Interesse umfasst, eine Markernucleotidsequenz umfasst, die in dem Polynucleotid außerhalb der Nucleotidsequenz der Nucleinsäure von Interesse lokalisiert ist.

15. Verfahren nach einem der Ansprüche 1 bis 14, **dadurch gekennzeichnet, dass** der DNA-Vektor ein bakterielles Plasmid ist.

16. Verfahren nach einem der Ansprüche 1 bis 15, **dadurch gekennzeichnet, dass** der DNA-Vektor ein in Bakterienzellen funktionelles Plasmid ist.

17. Verfahren nach einem der Ansprüche 1 bis 15, **dadurch gekennzeichnet, dass** der DNA-Vektor ein in humanen Zellen funktionelles Plasmid ist.

18. Verfahren nach einem der Ansprüche 1 bis 17, **dadurch gekennzeichnet, dass** der DNA-Vektor eine lineare doppelsträngige DNA ist.

19. Verfahren nach einem der Ansprüche 1 bis 18, **dadurch gekennzeichnet, dass** die in Stufe b) transfizierten Zellen aus Bakterienzellen bestehen.

20. Verfahren nach einem der Ansprüche 1 bis 18, **dadurch gekennzeichnet, dass** die in Stufe b) transfizierten Zellen aus nicht humanen Säugerzellen bestehen.

21. Verfahren nach einem der Ansprüche 1 bis 18, **dadurch gekennzeichnet, dass** die in Stufe b) transfizierten Zellen aus humanen Zellen, mit Ausnahme von humanen Keimzellen und humanen embryonalen Stammzellen bestehen.


### LISTE DE SEQUENCES

<110> CENTRE NATIONAL DE LA RECHERCHE SCIENTIFIQUE
<120> Procédé pour insérer un acide nucléique d'intérêt dans une cellule procaryote ou eucaryote par recombinaison homologue.
<130> N771PCT-CNRS
<140>
   <141>
<150> FR02134174
   <151> 2002-10-28
<160> 9
<170> PatentIn Ver. 2.1
<210> 1
   <211> 20
   <212> ADN
   <213> Séquence artificielle
<220>
   <223> Description de la séquence artificielle:amorce
<400> 1
   ggtacaactt gccaactggg 20
<210> 2
   <211> 20
   <212> ADN
   <213> Séquence artificielle
<220>
   <223> Description de la séquence artificielle : amorce
<400> 2
   ttgtcacgtc actcagctcc 20
<210> 3
   <211> 24
   <212> ADN
   <213> Séquence artificielle
<220>
   <223> Description de la séquence artificielle:amorce
<400> 3
   gccggccacg tgatttaaat acgt 24
<210> 4
   <211> 20
   <212> ADN
   <213> Séquence artificielle
<220>
   <223> Description de la séquence artificielle:amorce
<400> 4
   ctttcctgcg ttatcccctg 20
<210> 5
   <211> 20
   *<*212> ADN
   <213> Séquence artificielle
<220>
   <223> Description de la séquence artificielle:amorce
<400> 5
   tcgccctttg acgttggagt 20
<210> 6
   <211> 20
   <212> ADN
   <213> Séquence artificielle
<220>
   <223> Description de la séquence artificielle:amorce
<400> 6
   agcacttcca cctgatctcc 20
<210> 7
   <211> 23
   <212> ADN
   <213> Séquence artificielle
<220>
   <223> Description de la séquence artificielle:amorce
<400> 7
   gctcctgtgt tcttcatgct tgg 23
<210> 8
   <211> 20
   <212> ADN
   <213> Séquence artificielle
<220>
   <223> Description de la séquence artificielle:amorce
<400> 8
   gctggccttt tgctcacatg 20
<210> 9
   <211> 20
   <212> ADN
   <213> Séquence artificielle
<220>
   <223> Description de la séquence artificielle:amorce
<400> 9
   aaagggggat gtgctgcaag 20